# DEMANDE DE BREVET EUROPEEN

(11) **EP 4 295 870 A1**
(43) Date de publication de la demande: **27.12.2023**
(21) Numéro de dépôt: 23180737.1
(22) Date de dépôt: 21.06.2023
(51) Int. Cl.: A61L 27/20, A61L 27/36

(54) **BIOMATÉRIAU COMPRENANT UNE POUDRE DE COPRODUIT DE POISSON ET DU CHITOSAN, PROCÉDÉ DE PRÉPARATION ET UTILISATION EN TANT QU'IMPLANT POUR LA RECONSTRUCTION OSSEUSE**

(30) Priorité: 23.06.2022 FR 2206238
(71) Demandeur: Abyss Ingredients, 56850 Caudan (FR); Université de Rennes, 35042 Rennes Cedex (FR); Universite De Bretagne Sud, 56100 Lorient (FR)
(72) Inventeur: OUDADESSE, Hassane, 75010 PARIS (FR); LEFEUVRE, Bertrand, 75010 PARIS (FR); HAMROUNI, Nada, 75010 PARIS (FR); CORRE, Yves-Marie, 75010 PARIS (FR); GROHENS, Yves, 75010 PARIS (FR); SAULNIER, Benjamin, 75010 PARIS (FR); BOUVRET, Elodie, 75010 PARIS (FR); MOLINARO, Jérémy, 75010 PARIS (FR)
(74) Mandataire: Oak & Fox

(57) **Abrégé**

L'invention concerne un biomatériau comprenant de 70 à 90 %, de préférence de 80 à 85%, en poids d'une poudre d'au moins un co-produit de poissons choisi dans le groupe formé par les écailles de poissons et les arêtes de poissons et de 10 à 30 %, de préférence de 15 à 20%, en poids de chitosan ; la mise en forme de ce biomatériau sous forme d'implant, notamment par impression 3D par dépôt de fil fondu : et l'utilisation de l'implant pour la repousse osseuse, la reconstruction osseuse, et/ou le traitement de l'ostéoporose.

## Description

La présente invention se rapporte au domaine des biomatériaux et plus particulièrement des biomatériaux utilisables dans le domaine médical, plus précisément en tant qu'implants pour la repousse osseuse, la reconstruction osseuse et/ou le traitement de l'ostéoporose.

Suite à des accidents, opérations, une partie de la reconstruction osseuse s'effectue naturellement par la capacité du corps à se régénérer, cependant si la zone abimée est trop importante, une intervention est souvent nécessaire.

Les greffes osseuses : autogreffes, xénogreffes et hétérogreffes restent les voies principales de reconstruction osseuse. Cependant, ces méthodes ne sont pas toujours applicables notamment en cas de maladies. De plus, les xénogreffes et hétérogreffes sont soumises aux contraintes de compatibilité inhérentes au donneur de l'os d'une part et au site du receveur de l'os autologue d'autre part.

Face à ces difficultés, l'ingénierie tissulaire osseuse constitue une méthode prometteuse de réparation des os. En conséquence, ce domaine fait toujours l'objet d'abondantes recherches afin de proposer de nouveaux matériaux biomimétiques, aptes à constituer une matrice pour la régénération osseuse.

Le processus de guérison des lésions osseuses implique une série d'activités cellulaires complexes, notamment l'agrégation des cellules souches mésenchymateuses, la prolifération des ostéoblastes, des macrophages, et de la matrice extracellulaire. De nombreux facteurs jouent un rôle dans ce processus. Principalement, les ostéoblastes prolifèrent et se différencient pour former l'hydroxyapatite qui est le sédiment de minéralisation osseuse, et les facteurs de croissance sécrétés par diverses cellules régulent l'angiogenèse et favorisent l'apport d'oxygène et de nutrition.

En conséquence, les matériaux de réparation des défauts osseux doivent être non cytotoxiques, peu ou pas immunogènes, ils doivent présenter une interface propice à la croissance de l'adhésion cellulaire. De plus ces matériaux doivent assurer le remplacement de l'os (endommagé) par le nouvel os (issu de la repousse osseuse) dans un temps déterminé.

Il demeure toujours un besoin de nouveaux matériaux répondant à ces exigences, qui soient compatibles avec les prix du marché et relativement faciles à être mis en forme pour pouvoir être utilisés en tant qu'implant.

Les biomatériaux selon la présente invention comprenant une poudre de co-produits de poissons et du chitosan répondent à ce besoin.

### Art antérieur

Les principaux biomatériaux utilisés comme substituts osseux sont les « céramiques bioactives » et les « verres bioactifs ».

Il est aussi connu d'utiliser des biomatériaux à base d'hydroxyapatite de synthèse ou de phosphate de calcium d'origine naturelle dans lequel l'hydroxyapatite utilisée est extraite par des techniques d'hydrolyse alcaline ou enzymatique ou encore par calcination en vue d'obtenir, selon le procédé choisi, de l'hydroxyapatite sous forme de poudre de l'ordre du micromètre ou du nanomètre, la granulométrrie étant choisie en fonction de leur utilisation finale.

A titre d'exemple, la demande de brevet CN112121228 A décrit un implant pour le remplissage des cavités osseuses comprenant des nanofibres à base d'un réseau réticulé de chitosan et d'acide polylactique à la surface desquelles des nanoparticules d'hydroxyapatites sont formées par voie sol-gel.

On observe cependant que les biomatériaux à base d'hydroxyapatite ne présentent pas toujours des propriétés mécaniques suffisantes pour pouvoir être utilisés pour la reconstruction des os porteurs.

Par ailleurs, les biomatériaux formés à partir de poudres issues de co-produits de poissons présentent les avantages liés aux produits naturels en termes de biocompatibilité, de disponibilité de la ressource. Néanmoins ces biomatériaux denses comprennent une partie inorganique et une partie organique et cette structure complexe fait qu'il n'est pas toujours évident de les associer avec d'autres composants et d'obtenir de nouveaux matériaux stables pendant leur stockage, avant implantation.

De manière inattendue et avantageuse, les inventeurs ont montré que l'utilisation d'un biomatériau particulier comprenant de la poudre de co-produits de poissons et du chitosan permet la préparation d'un matériau d'implant stable pour le comblement des défauts osseux, la régénération osseuse et l'ingénierie tissulaire de l'os.

### Exposé de l'invention

Un premier objet de l'invention vise un biomatériau comprenant, par rapport au poids total du biomatériau, de 70 à 90 %, de préférence de 80 à 85%, en poids d'une poudre d'au moins un co-produit de poissons choisi dans le groupe formé par les écailles de poissons et les arêtes de poissons et de 10 à 30 % en poids, de préférence de 15 à 20% en poids de chitosan.

Un deuxième objet de l'invention vise un procédé de fabrication du biomatériau selon l'invention comprenant les étapes suivantes, dans cet ordre :
- disposer d'une poudre d'au moins un co-produit de poissons choisi dans le groupe formé par les écailles de poissons et les arêtes de poissons, ladite poudre présentant une granulométrie allant de 40 à 315 microns ;
- disposer de chitosan ;
- préparer un mélange comprenant de 70 à 90 %, de préférence de 80 à 85%, en poids de poudre d'au moins un co-produit de poissons et de 10 à 30 % en poids, de préférence de 15 à 20% en poids de chitosan ;
- agiter le mélange ;
- sécher le précipité obtenu ;
- récupérer le biomatériau obtenu.

Un troisième objet de l'invention vise une composition comprenant un biomatériau selon l'invention et au moins un agent choisi parmi les médicaments en particulier les antibiotiques, les bisphosphonates, les polymères thermoplastiques en particulier choisis parmi l'acide polylactique (PLA), l'acide poly (lactique-co-glycolique) (PLGA), le polybutylène succinate (PBS), les polyhydroxyalcanoates (PHA), la polycaprolactone (PCL), le polybutylène-co-adipate téréphtalate (PBAT).

Un quatrième objet de l'invention vise un implant comprenant un biomatériau selon l'invention ou obtenu suivant le procédé selon l'invention ou la composition selon l'invention, et son utilisation pour la repousse osseuse, la reconstruction osseuse, et/ou le traitement de l'ostéoporose.

Un cinquième objet de l'invention vise un procédé de fabrication de l'implant selon l'invention comprenant avantageusement une étape d'impression 3D du biomatériau ou de la composition. En particulier le procédé de fabrication de l'implant selon l'invention comprend les étapes suivantes, dans cet ordre,
- mélange du biomatériau selon l'invention, avec au moins un matériau thermoplastique choisi parmi l'acide polylactique, l'acide poly (lactique-co-glycolique), le polybutylène succinate, les polyhydroxyalcanoates, la polycaprolactone, le polybutylène-co-adipate téréphtalate ; au moins un agent porogène et éventuellement au moins un agent choisi parmi les médicaments en particulier les antibiotiques, les bisphosphonates ;
- mise en forme du mélange par impression 3D par dépôt de fil fondu,
- élimination des agents porogènes de préférence par lixiviation,
- éventuellement séchage de l'implant obtenu.

Les co-produits de poissons tels que les écailles de poissons et les arêtes de poissons contiennent une partie inorganique comprenant un phosphate de calcium particulier : l'hydroxyapatite, associée à d'autres minéraux mineurs et à l'état de traces ainsi qu'une partie organique comprenant du collagène.

Sans vouloir être lié à une quelconque théorie, il semble que la combinaison d'une poudre de co-produit de poissons, comprenant de l'hydroxyapatite ainsi que d'autres minéraux et du collagène avec du chitosan permet l'obtention d'un biomatériau présentant des propriétés particulièrement avantageuses pour une utilisation en tant que matériau d'implant osseux. En particulier le biomatériau présente une bonne biocompatibilité : aucun rejet ni inflammation n'ont été observés, il permet la différenciation et la prolifération des cellules ostéoblastiques et donc favorise l'ostéogénèse. Il semble que les quantités d'hydroxyapatite, de collagène et de minéraux (oligoéléments) soient particulièrement avantageuses pour les applications visées.

En effet l'utilisation de co-produits de poissons conformément à l'invention permet de conserver un équilibre entre les différents composants organiques et inorganiques. Or cet équilibre a pu être rompu dans les applications de l'art antérieur dans lesquelles de l'hydroxyapatite extraite d'une source naturelle est utilisée.

### Avantages

Le biomatériau selon l'invention permet de préparer un implant de forme adaptée au défaut osseux à réparer. Le biomatériau selon l'invention est non cytotoxique et biocompatible, il peut être implanté de façon conventionnelle et favorise l'ostéogénèse au niveau du site d'implantation : en effet il favorise l'adhésion des cellules osseuses. Il est également biorésorbable puisqu'il est progressivement remplacé par du tissu osseux à mesure de la progression de la repousse osseuse. Sa préparation est aisée et peut être effectuée au moyen de dispositifs courants dans le commerce à un prix raisonnable.

Chez des sujets, humains ou animaux, atteints d'ostéoporose, le biomatériau selon l'invention permet de réduire l'ostéoporose.

### Description des figures

Fig.1 présente l'analyse structurale par diffraction des rayons X (DRX) de l'implant de l'exemple 2 avant et après implantation, en comparaison avec le diffractogramme de l'os de ratte saine.
Fig.2 présente l'analyse structurale par diffraction des rayons X (DRX) de l'implant de l'exemple 3 avant et après implantation, en comparaison avec le diffractogramme de l'os de ratte saine.
Fig.3 présente les résultats du dosage de la phosphatase alcaline (PAL) entre 15 et 90 jours chez des sujets où les implants des exemples 2 et 3 ont été insérés, par rapport aux témoins.
Fig.4 présente la coupe d'un condyle fémoral d'une ratte saine (à gauche) et la coupe d'un condyle fémoral d'une ratte ovariectomisée (à droite).
Fig.5 présente les coupes de condyles fémoraux d'une ratte après implantation depuis 90 jours d'un implant selon l'exemple 2 (à gauche) et d'un implant selon l'exemple 3 (à droite), les rattes ayant été ovariectomisées 2 mois auparavant.
Fig.6 présente une comparaison entre les analyses histologiques à 90 jours d'un implant selon l'exemple 3 (à droite) vs un implant comparatif exemple 4 sans chitosan (à gauche).

D'autres aspects, avantages, propriétés de la présente invention sont présentés dans la description et les exemples qui suivent.

### Description détaillée de l'invention

### Biomatériau

Comme déjà décrit, le biomatériau selon l'invention comprend de 70 à 90 %, de préférence de 80 à 85%, en poids, d'une poudre d'au moins un co-produit de poissons choisi dans le groupe formé par les écailles de poissons et les arêtes de poissons et de 10 à 30 % en poids, de préférence de 15 à 20% en poids de chitosan.

L'actif principal du biomatériau comprend une poudre d'au moins un co-produit de poissons et du chitosan.

Avantageusement le ratio massique entre la poudre de co-produit de poissons et le chitosan va de 4,5 à 5,5, de préférence de 4,7 à 5,1.

### Poudre de coproduits de poisson

Les co-produits de poissons utilisables sont choisis dans le groupe formé par les écailles de poissons, les arêtes de poissons et leurs mélanges. Les arêtes de poissons peuvent être obtenues à partir de toutes espèces de poissons.

En revanche, les écailles de poissons sont généralement choisies parmi les écailles de sardines, saumons, carpes, tilapias, bars, dorades et de manière préférée les sardines.

Avantageusement, les écailles de poissons sont utilisées sans traitement, notamment elles ne sont pas décellularisées.

Le co-produit de poissons peut également comprendre un mélange d'écailles de poissons et d'arêtes de poissons, les mélanges en toutes proportions sont utilisables.

Avantageusement, la poudre de co-produit de poissons présente une granulométrie allant de 40 à 315 microns (10⁻⁶ mètre).

Il semble que le choix de cette granulométrie particulière permet une bonne intéraction entre les différents composants du mélange à savoir la partie inorganique, le collagène et le chitosan qui conduit à un composite stable, homogène et compaptible avec une mise en forme adéquate pour une utilisation en tant d'implant osseux.

La poudre utilisée selon l'invention est obtenue à partir de co-produits de poissons choisis dans le groupe formé par les écailles de poissons et les arêtes de poissons. Ces co-produits de poissons comprennent comme composant majoritaire un phosphate de calcium particulier : l'hydroxyapatite (HA) de formule chimique [Ca₁₀(PO₄)₆(OH)₂] qui présente un réseau hexagonal.

Ainsi, le co-produit de poissons utilisé selon l'invention comprend du calcium et du phosphore selon un rapport calcium/ phosphore Ca/P en poids allant de 1,8 à 2,5 de préférence allant de 2,0 à 2,5.

Dans le cas particulier des co-produits de poissons utilisés selon l'invention, l'hydroxyapatite est associée d'une part à d'autres minéraux dont le principal est le magnésium Mg et d'autre part à du collagène.

Le co-produit de poissons utilisé selon l'invention comprend du collagène en une quantité allant de 20% à 40 % et de préférence allant de 20% à 35% en poids par rapport au poids total du co-produit de poissons.

Le co-produit de poissons utilisé selon l'invention comprend aussi du magnésium (Mg) en une quantité allant de 0,30% à 0,60 %, et de préférence allant de 0,44% à 0,46% en poids par rapport au poids total du co-produit de poissons.

Le co-produit de poissons utilisé selon l'invention comprend également les éléments suivants en poids par rapport au poids total du co-produit de poissons : du zinc (Zn) en une quantité allant de 0,010% à 0,011%, du strontium (Sr) en une quantité en poids par rapport au poids total du co-produit de poissons allant de 0,032% à 0,033 %.

Les poudres de co-produits de poisson utilisables selon l'invention sont disponibles sur le marché, elles sont notamment commercialisées par la société Abyss Ingrédients sous les dénommination Calcilyss et Phosphymer.

Selon une autre alternative, la poudre de co-produits de poisson utilisable selon l'invention peut être préparée selon le procédé de préparation suivant.

Ainsi la présente invention vise aussi un procédé de fabrication de la poudre de co-produit de poissons en mettant en oeuvre les étapes suivantes, dans cet ordre :
- disposer d'au moins un co-produit de poissons choisi parmi les écailles de poissons et les arêtes de poissons,
- placer le co-produit de poissons dans un bain d'eau à une température allant de 50°C à 100°C pendant une durée allant de 5 à 120 minutes,
- éventuellement égoutter le co-produit de poissons,
- sécher le co-produit,
- broyer le co-produit de manière à obtenir une poudre de granulométrie allant de 40 à 315 microns,
- récupérer la poudre de co-produit de poissons obtenue.

Avantageusement, lorsque des arêtes de poissons sont utilisées pour l'obtention du co-produit de poissons, alors le procédé de fabrication de la poudre de co-produit de poissons peut comprendre une étape préalable de séparation mécanique de la chair de poisson collée aux arêtes.

Avantageusement, lorsque des écailles de poissons sont utilisées pour l'obtention du co-produit de poissons, alors le procédé de fabrication de la poudre de co-produit de poissons peut comprendre une étape préalable de récupération mécanique des écailles.

### Chitosan

Le chitosan est un biopolymère naturel d'origine marine consistant en des liaisons d'unités ß-(1 → 4)-2-acétamido-d-glucose et ß-(1 → 4)-2-amino-d-glucose, obtenu par déacylation de la chitine.

Selon un mode préféré, le chitosan utilisé dans la présente invention présente un degré de désacétylation (DD) de 75% et un poids moléculaire compris entre 190 et 310 kDa.

Généralement, le chitosan est sous forme de poudre de particules de taille inférieure à 1000 microns et de densité allant de 0,45 à 0,65 g/cm³.

### Utilisation

La présente invention vise un implant comprenant un biomatériau selon l'invention ou obtenu suivant le procédé selon l'invention ou encore comprenant une composition selon l'invention et son utilisation pour la repousse osseuse, la reconstruction osseuse, le comblement osseux et/ou le traitement de l'ostéoporose.

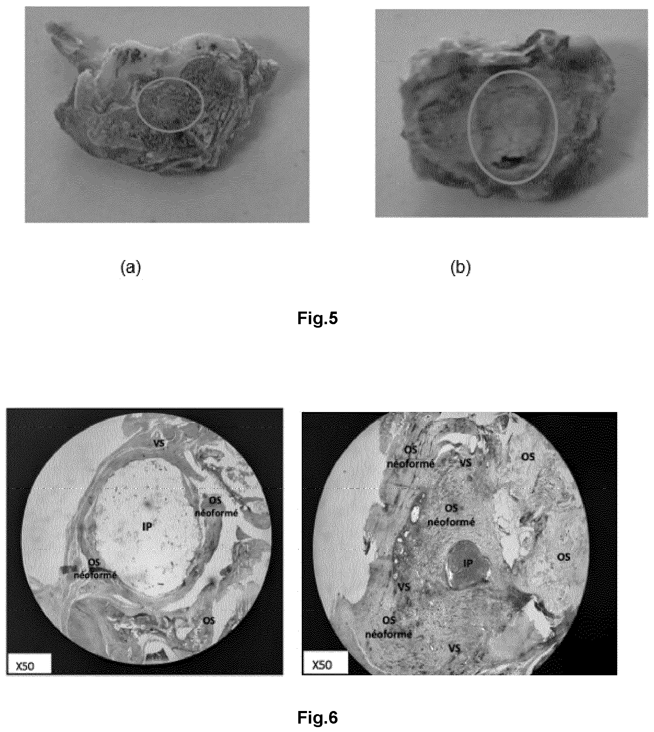

Comme il apparait sur les photos des figures 5 et 6, l'implant joue un rôle de matrice dans la repousse osseuse et la reconstruction osseuse. Il est avantageusement biodégradable, biorésorbable puisqu'il est progressivement remplacé par le tissu osseux à mesure de la progression de la repousse osseuse c'est-à-dire de la formation du tissu néo-formé. L'implant permet donc de combler le défaut osseux à 100%.

Selon une première variante, l'implant comprend, en poids par rapport au poids total de l'implant, de 90 à 100 % d'un biomatériau selon la présente invention et de 0 à 10 % d'au moins un agent choisi parmi les bisphosphonates et les médicaments, en particulier les antibiotiques. Selon cette première variante, l'implant peut-être essentiellement constitué du biomatériau selon la présente invention.

Selon cette première variante, l'implant est utilisé sous forme de poudre libre ou compactée.

L'implant sous forme de poudre peut être avantageusement utilisé en chirurgie cranio-maxillo faciale.

Comme montré au paragraphe 2.3 ci-dessous, l'implant selon cette première variante permet une ostéointégration rapide qui est particulièrement souhaité pour les sujets jeunes, et chez les sportifs.

Selon une deuxième variante, l'implant comprend, en poids par rapport au poids total de l'implant, de 30 à 50 % d'un biomatériau selon la présente invention, de 50 à 70% d'un matériau thermoplastique choisi dans le groupe formé par l'acide polylactique (PLA), l'acide poly (lactique-co-glycolique) (PLGA), le polybutylène succinate (PBS), les polyhydroxyalcanoates (PHA), la polycaprolactone (PCL), le polybutylène-co-adipate téréphtalate (PBAT) et de 0 à 10 % d'au moins un agent choisi parmi les médicaments, en particulier les antibiotiques et les bisphosphonates.

Selon cette deuxième variante, l'implant est avantageusement obtenu par une méthode de fabrication additive de type impression 3D par dépôt de fil fondu ou FFF pour « Fused Filament Fabrication ». La mise en oeuvre de cette variante comprend l'étape initiale de formulation par mélange en voie fondue à l'aide d'une extrudeuse bivis du biomatériau sous forme de poudre, d'une matrice thermoplastique biocompatible et d'au moins un agent porogène. La formulation est ensuite transformée en filaments conformes aux standards de la technologie dépôt de fil fondu à l'aide d'une extrudeuse monovis équipée d'une ligne de filage de profilés. Le filament, généralement de diamètre 1,75mm ou 2,80mm, est ensuite mis en forme par impression 3D puis il est soumis à un traitement d'élimination des agents porogènes, notamment une lixiviation aqueuse, éventuellement suivi d'un étuvage pour séchage.

Lors de la mise en oeuvre de la méthode de fabrication additive, sont utilisés de 20 à 40 % en poids et de préférence de 25 à 35% en poids de matériau thermoplastique, de 30% à 65 % en poids et de préférence de 42 à 57% d'au moins un agent porogène et de 15 à 25 % en poids du biomatériau coproduit selon la présente invention.

Généralement 2 à 3 agents porogènes organiques ou inorganiques sont utilisés. Les agents porogènes, hydrosolubles permettent de créer une structure poreuse suite à leur élimination de la formulation, généralement par une étape de lixiviation en milieu aqueux.

Généralement les agents porogènes organiques sont des polymères hydrosolubles choisis parmi le polyvinyalcool (PVA) et les polyéthylèneglycol (PEG) à poids moléculaire compris entre 1000 g/mol et 15 000 g/mol et de préférence entre 4000 g/mol et 10 000 g/mol.

A titre d'agent porogène inorganique est généralement utilisé le chlorure de sodium (NaCl) sous forme de poudre de granulométrie allant de 10 à 700 µm et de préférence de 100 à 400 µm.

Comme montré au paragraphe 2.3 ci-dessous, cette deuxième variante de l'implant permet une ostéointégration plus lente, encore incomplète 3 mois après implantation, durée correspondant à la fin de l'étude.

Les exemples qui suivent visent à illustrer l'invention sans en limiter la portée.

### Exemples

### A- Préparation de poudres de co-produit de poissons

### 1. Poudre d'écailles de sardines

Les écailles de sardines collectées sont placées dans un bain d'eau à 90°C pendant 30 minutes, elles sont ensuite égouttées avant d'étre congelées.

Après décongélation, 100 g d'écailles sont placées à l'étuve pendant 60 heures à 94°C, le produit sec est ensuite broyé puis tamisé afin d'obtenir une poudre d'écailles de sardines de granulométrie inférieure à 315 microns. On obtient 46 g de poudre.

### 2. Poudre d'arêtes de poissons blancs

Les arêtes de poissons blancs de la famille des gadidés sont collectées puis soumises à un traitement destiné à séparer la chair des arêtes : séparation mécanique suivie d'un passage dans un bain d'eau chaude à 90°C pendant 30 minutes puis centrifugation.

Les arêtes ainsi traitées sont égouttées sur tapis puis congelées.

Après décongélation, 100 g d'arêtes sont placées à l'étuve pendant 60 heures à 94°C, le produit sec est ensuite broyé puis tamisé afin d'obtenir une poudre d'écailles de sardines de granulométrie inférieure allant de 40 à 315 microns. On obtient 43 g de poudre.

Le tableau 1 présente les compositions chimiques des poudres d'écailles et d'arêtes obtenues ci-dessus. Les quantités des différents éléments sont obtenues par analyse par spectrométrie d'émission atomique à plasma à couplage inductif (ICP-OES) à l'aide d'un appareil iCAP 7000 (ThermoFisher Scientific), à l'exception du taux de protéines collagéniques (collagène) qui est obtenu par dosage de l'azote.

**[Tableau 1]**

| | Poudre d'écaille de sardines | Poudre d'arête de poissons blancs | Hydroxy-apatite de synthèse | Os mature (rat) |
|---|---|---|---|---|
| Collagène (% massique) | 29,3 | 24,2 | 0 | Non mesuré |
| Ca (% massique) | 25,18 | 27,9 | 35,98 | 20,01 |
| P (% massique) | 12,27 | 13,6 | 16,86 | 9,46 |
| Rapport Ca/P (% massique) | 2,05 | 2,05 | 2,13 | 2,12 |
| Mg (% massique) | 0,44 | Non mesuré | 0,17 | 0,30 |
| Zn (% massique) | 0,011 | Non mesuré | 0,004 | 0,033 |
| Sr (% massique) | 0,032 | Non mesuré | 0,014 | 0,004 |
| Na(% massique) | 0,21 | Non mesuré | 0,48 | 0,475 |

La poudre d'écailles utilisée selon l'invention présente un rapport Ca/P proche de celui de l'os sain mature. En outre, la poudre d'écaille comprend les principaux minéraux présents dans l'os mature en des quantités significatives.

### B. Préparation des biomatériaux et caractérisation

### Exemple 1 : préparation d'un implant sous forme de poudre

24,9 g (83%pds) de poudre d'écailles de sardines ont été dispersé dans 50 millilitres d'acide acétique. Ensuite la solution de poudre de co-produit a été introduite goutte à goutte dans la solution du chitosan préalablement préparée (5,1g chitosan (17% en poids), dans 200 ml d'acide acétique). Le mélange obtenu a été agité pendant 24 heures en utilisant une agitation magnétique à 1200 (tour /minute). Après élimination du surnageant, le mélange a été congelé puis solidifié dans de l'azote liquide pendant 10 minutes et enfin lyophilisé à -60°C, pendant 24heures pour éliminer les solvants. Par la suite, le biomatériau obtenu a été immergé dans la soude à 0,2M pendant 2 heures, puis lavé avec de l'eau distillée pour neutraliser totalement les radicaux d'acide acétique. Le biomatériau obtenu a été mélangé à température ambiante avec 20% en poids de polyvinylalcool, puis à nouveau congelé et lyophilisé pendant 24 heures pour évacuer totalement l'eau. On obtient ainsi 0,068 g d'un implant sous forme de poudre.

### Exemple 2 : préparation d'un implant par compactage

Le biomatériau obtenu à l'exemple 1 a été inséré dans un moule en laiton comportant deux compartiments et deux pistons. Le compactage de la poudre est réalisé en exerçant une force de 15 kg à l'aide des pistons afin d'obtenir des cylindres de 3 mm par 3 mm. Un implant de 0,068 g sous forme d'une poudre compactée comprimé de 3 mm de diamètre et 3 mm de hauteur a été obtenu. Cet implant est ensuite utilisé pour l'implantation dans des condyles fémoraux pour l'étude in vivo.

### Exemple 3 : préparation d'un implant par fabrication additive

Une formulation biocomposite thermoplastique est préparée par mélange en voie fondue à l'aide d'une extrudeuse bivis co-rotative FSCM21 de marque TSA (Luisago, Italie) équipée de vis de diamètre 21 mm de rapport L :D=40 et d'une filière d'extrusion de 3 joncs de 4mm de diamètre. L'ensemble des composants de la formulation est introduit en trémie principale de l'extrudeuse bivis à l'aide de deux doseurs gravimétriques DDW-MD3-DDSR20 et DDW-M-DSR28 de marque Brabender (Duisburg, Allemagne). Le premier doseur est dédié au dosage d'un pré-mélange des éléments sous forme de poudres incluant le co-produits de poissons, le chitosan et les agents porogènes constitués d'un polymère hydrosoluble de type PEG 8000 et d'un sel hydrosoluble de type NaCl. Le second doseur est dédié au dosage du biopolymère de type acide polylactique (PLA) se présentant sous forme de granulés. Le débit cumulé de ces doseurs est fixé à 2,4 kg/H. Le profil des températures sur les huit zones de chauffe est le suivant : Z₁₋₂=160°C, Z₃=175°C et Z₄₋₈=180°C. La vitesse de rotation des vis est fixée à 400 RPM. Les joncs sont refroidis en sortie de filière à l'air ambiant sur un tapis de convoyage à bande puis granulés à l'aide d'un granulateur à couteaux rotatifs.

La composition de la formulation mise en oeuvre est donnée dans le tableau 2 ci-dessous.

**[Tableau 2]**

| | PLA | PEG 8000 | NaCl | Co-Produit poisson | Chitosan |
|---|---|---|---|---|---|
| Densité (g/L) | 1,25 | 1,20 | 2,16 | 1,82 | 1,40 |
| Ratio massique | 30 | 8 | 42 | 16,6 | 3,4 |
| Ratio Volumique | 38.94 | 10.82 | 31.54 | 14.76 | 3,94 |

La formulation est ensuite transformée en filaments de diamètre 1,75mm à l'aide d'une extrudeuse monovis de marque SCAMEX (Saumur, France) de diamètre 20 mm et ratio L/D=20 équipée d'une filière annulaire de diamètre 2mm et d'une ligne de tirage, contrôle dimensionnel et bobinage. Le profil des températures sur les quatre zones de chauffe est le suivant : Z₁₋₂=180°C, Z₃₋₄=170°C. La vitesse de rotation de la vis est fixée à 15 RPM et la vitesse de la ligne de tirage est fixée à 3,8m/min.

Le filament produit est ensuite utilisé en impression 3D FFF au moyen d'une imprimante µ-Delta Reworks de la marque Emotiontech (Toulouse, France) équipée d'une buse de 0,8 mm de diamètre. L'implant consiste en un cylindre de 3mm de diamètre et de 3mm de hauteur. La géométrie 3D de l'implant est modélisée à l'aide du logiciel Solidworks de la marque Dassault Systèmes. Le format de sortie du fichier 3D est de type STL. La programmation de l'impression à partir du fichier 3D STL est réalisée à l'aide du logiciel Repetier Host de la marque Hot-World GmbH & Co (Willich, Allemagne) utilisant l'argorithme de tranchange Slic3r (Opensource). L'impression est réalisée à une température de buse de 225°C et une température de plateau d'impression de 75°C. La hauteur de couche est fixée à 0,6 mm et la largeur de couche est fixée à 0,9mm. La vitesse d'impression est fixée à 6mm/s.

L'implant obtenu par impression 3D FFF est ensuite post-traité par lixiviation aqueuse afin d'éliminer les agents porogènes. Pour se faire, l'implant est immergé dans de l'eau déminéralisée et est soumis à un traitement en bain ultrasons à une température de 45 °C pendant 100 min. Un rinçage à l'eau déminéraliséé est ensuite réalisé. L'implant est soumis à 5 cycles de ce post-traitement avant un étuvage sous vide à 45°C pendant 96 heures.

### Exemple 4 comparatif : implant comparatif obtenu à partir de poudre par fabrication additive

Un implant sans chitosan est obtenu selon le même procédé que celui présenté à l'exemple 3 à partir de la formulation du tableau 3 ci-dessous.

**[Tableau 3]**

| | PLA | PEG 8000 | NaCl | Co-Produit poisson |
|---|---|---|---|---|
| Densité (g/L) | 1.25 | 1.20 | 2.16 | 1.82 |
| Ratio massique | 30 | 8 | 42 | 20 |
| Ratio Volumique | 39.30 | 10.92 | 31.84 | 17.95 |

### C.Tests biologiques

### 1.Cytotoxicité

La cytotoxicité a été mesurée par MTT. Le MTT est un sel de tétrazolium 3-[4,5 dimethylthiazol-2-yl]-2,5-diphenyl tetrazolium bromide (Sigma-Aldrich Corporation, St. Louis, USA), de couleur jaune. En présence de cellules vivantes métaboliquement actives, le MTT est clivé par des succinate déshydrogénases de la chaîne respiratoire mitochondriale pour former des cristaux de formazan violets. Après solubilisation des cristaux avec du DMSO (Dimethyl Sulfoxide), le milieu passe du jaune au violet, et l'absorbance de la solution colorée obtenue est mesurée à 570 nm par spectrophotométrie. L'intensité de la coloration est proportionnelle à la quantité de cellules vivantes et donne une indication du pourcentage de la viabilité cellulaire. Le test au MTT permet de mesurer l'activité métabolique des cellules et donc de quantifier l'effet cytotoxique de coproduits après 72 heures de contact.

La cytotoxicité des biomatériaux des exemples 2 et 3 *in vitro* a été effectuée sur 3 types de cellules impliquées dans la régénération osseuse : des fibroblastes (L929), des ostéoblastes SaOS2 qui sont des cellules d'ostéosarcome humain (SaOS2) et des cellules de lignée cellulaire osseuses (LIG). Pour les deux biomatériaux on observe une bonne tolérance et une absence de toxicité.

Les biomatériaux selon l'invention sont biocompatibles.

### 2.Tests in vivo

Pour évaluer la formation osseuse après implantation du biomatériau selon l'invention le protocole suivant a été mis en place :

### Protocole expérimental

Des rattes Wistar âgées de 6 mois et de poids moyen 230 ± 20 g ont été réparties au hasard suivant les six groupes (a-f) ci-dessous pour évaluations physico-chimiques et biologiques :
- groupe a : témoin (-) : 8 rattes saines (aucun traitement) ;
- groupe b : témoin (+) : 6 rattes ovariectomisées ;
- groupe c : gap vide : 6 rattes ovariectomisées avec création d'un défaut osseux sans insertion d'un implant ;
- groupe d : insertion de l'implant de l'exemple 2 dans le gap créée dans les 2 fémurs par rattes : 12 rattes implantées ;
- groupe e : insertion de l'implant de l'exemple 3 dans le gap créé dans les 2 fémurs par rattes : 16 rattes implantées ;
- groupe f : comparatif : insertion de l'implant comparatif de l'exemple 4 obtenu à partir de poudre de co-produit de poisson par fabrication additive dans le gap créée dans les 2 fémurs par rattes : 16 rattes implantées.

Les conditions de l'élevage en animalerie conformes aux recommandations éthiques étaient les suivantes :
- température : ambiante varie entre 23 et 30 °C ;
- lumière : le cycle d'intensité lumineuse requis pour les animaux de laboratoire est de 12h/12h (cycle jour/nuit) ;
- les animaux ont libre accès à l'eau et à la nourriture qui était équilibrée d'un point de vue énergétique.

Après acclimatation des rattes aux conditions de l'animalerie, les implants ont été insérés dans les 2 condyles fémoraux de chaque ratte (groupes d-f) puis des prélèvements des fémurs implantés ont été effectués au bout de 15 jours, 30 jours, 60 jours et 90 jours.

Les analyses physicochimiques et évaluations biologiques suivantes ont été effectuées.

### 2.1 Analyses DRX

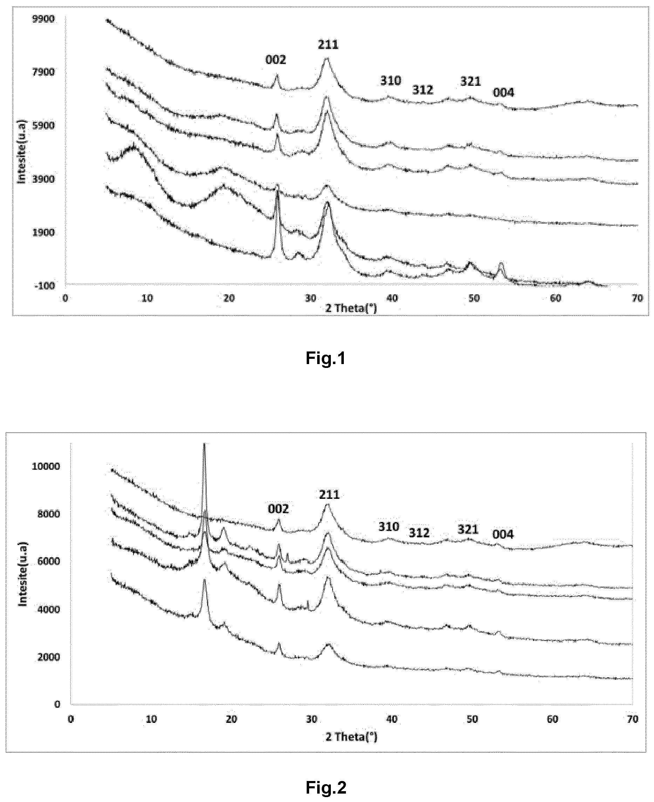

A la figure 1 est présentée l'analyse structurale par diffraction des rayons X (DRX) de l'implant de l'exemple 2 (groupe d) avant et après implantation. Un diffractomètre PANalytical X'Pert PRO a été utilisé. Les mesures ont été effectuées sur une plage allant de 5 à 70 ° (2θ). Une acquisition de 2 heures par échantillon a été réalisée. Les diagrammes DRX obtenus sont par la suite analysés et comparés à un os de ratte saine.

La numérotation des courbes suivantes correspond aux courbes de bas en haut :
la courbe 1 correspond aux raies de la poudre d'écailles de sardines de préparée au paragraphe A (référence) ;
la courbe 2 correspond aux raies de l'implant au bout de 15 jours (J15) ;
la courbe 3 correspond aux raies de l'implant au bout de 30 jours (J30);
la courbe 4 correspond aux raies de l'implant au bout de 60 jours (J60) ;
la courbe 5 correspond aux raies de l'implant au bout de 90 jours (J90);
la courbe 6 correspond aux raies de l'os de ratte saine.

Sur la courbe 2 (J15), on observe deux raies à 10° et 20° caractéristiques du chitosan, ces raies témoignent de l'incorporation de la poudre de co-produit de poisson dans la matrice du chitosan. Ces raies ne sont plus clairement observées sur les courbes 3 à 5, on en déduit que le chitosan ne reste pas au site d'implantation. Le chitosan ainsi libéré peut participer au traitement local de l'ostéoporose créée par ovariectomie.

A la figure 2 est présentée l'analyse structurale par diffraction des rayons X (DRX) du biomatériau de l'exemple 3 (groupe e) avant et après implantation. La numérotation des courbes suivantes correspond aux courbes de bas en haut :
la courbe 1 correspond aux raies de l'implant au bout de 15 jours (J15) ;
la courbe 2 correspond aux raies de l'implant au bout de 30 jours (J30);
la courbe 3 correspond aux raies de l'implant au bout de 60 jours (J60) ;
la courbe 4 correspond aux raies de l'implant au bout de 90 jours (J90);
la courbe 5 correspond aux raies de l'os de ratte saine.

Les figures 1 et 2 montrent la croissance d'une apatite biologique proche de la composition de l'os sain : en effet les courbes des implants à J60 et J90 sont très proches de celles de l'os sain, elles traduisent aussi une maturation osseuse progressive

L'évolution des minéraux des implants des exemples 2 et 3 a également été déterminée par spectrométrie d'émission atomique à plasma à couplage inductif (ICP-OES) à l'aide d'un appareil iCAP 7000 (ThermoFisher Scientific). Ces valeurs sont présentées dans le tableau 4.

**[Tableau 4]**

| | | Ca (% pds) | P (% pds) | Rapport Ca/P (% pds) | Mg (% pds) | Zn (% pds) | Sr (% pds |
|---|---|---|---|---|---|---|---|
| Os ratte saine | J0 | 21,51 | 9,66 | 2,23 | 0,30 | 0,031 | 0,005 |
| | J90 | 22,83 | 10,01 | 2,28 | 0,29 | 0,033 | 0,005 |
| Os ratte ovacte rio misée | J0 | 21,33 | 9,53 | 2,23 | 0,31 | 0,029 | 0,005 |
| | J90 | 20, 93 | 9,34 | 2,24 | 0,32 | 0,029 | 0,007 |
| Implant ex. 2 | J0 | 23,71 | 9,58 | 2,47 | 0,31 | 0,015 | 0,027 |
| | J90 | 20,88 | 9,14 | 2,28 | 0,33 | 0,026 | 0,004 |
| Implant ex. 3 | J0 | 11,6 | 3,4 | 3,41 | 0,126 | 0,003 | 0,014 |
| | J90 | 13,97 | 6,272 | 2,23 | 0,227 | 0,007 | 0,008 |

Il ressort du tableau 4 que le rapport Ca/P massique évolue dans le temps pour se rapprocher d'une valeur correspondant à celle de l'os de ratte saine : l'équilibre phosphocalcique n'est pas perturbé et les profils des minéraux ne sont pas altérés.

### 2.2 Dosage de la PAL

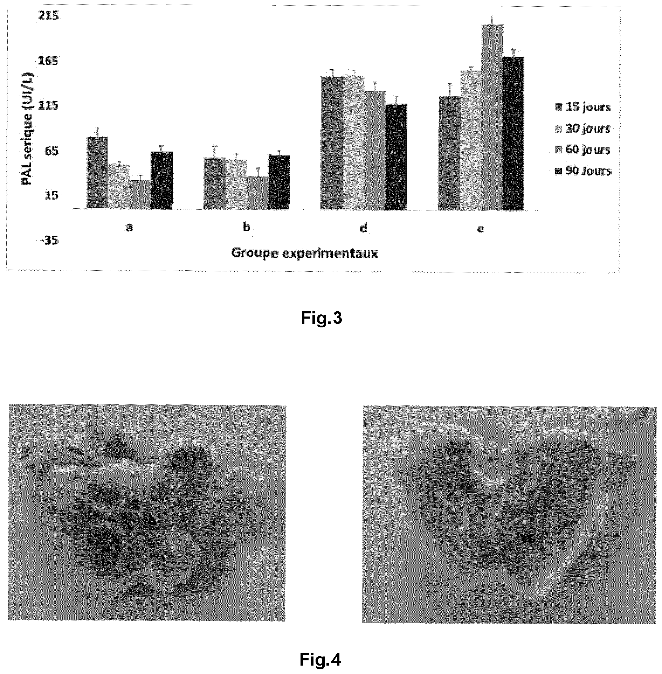

La figure 3 présente les résultats du dosage de l'enzyme phosphatase alcaline (PAL) entre 15 et 90 jours dans les groupes où les implants des exemples 2 et 3 ont été insérés, respectivement groupes d et e, par rapport au groupe a (témoin négatif) et au groupe b (témoin positif).

La PAL est un marqueur du remodelage osseux, cette enzyme présente dans les ostéoblastes (cellules osseuses) assure le dépôt des cristaux d'hydroxyapatite, elle est aussi associée à une augmentation de l'activité des ostéoblastes. Le dosage de la PAL a été effectué par la méthode cinétique à l'aide de l'automate BECKMAN COULTER. Les enzymes phosphatases alcalines, en présence de diéthanolamine à pH=10.3, agent transphorylant, hydrolysent le nitrophényl -4-phosphate. L'activité catalytique de la PAL est déterminée en mesurant la vitesse d'apparition de l'un des produits de la réaction, le nitro-4-phenol qui présente un maximum d'absorption à 410 nm. Les valeurs sont obtenues en UI/L. La figure 3 montre que les résultats obtenus pour les groupes d et e sont nettement supérieurs aux témoins a et b ce qui confirme une activation de la formation osseuse.

### 2.3 Aspect des condyles fémoraux au bout de 90 jours d'implantation

Sur la figure 4, la photographie de gauche présente la coupe d'un condyle fémoral d'une ratte saine et la photographie de droite présente la coupe d'un condyle fémoral d'une ratte ovariectomisée. Les coupes histologiques (figure 4) ont été réalisées avec un grossissement x50. Sur la photographie de gauche, le condyle fémoral de ratte saine présente un réseau dense de trabécules, alors que sur la photographie de droite, le condyle fémoral d'une ratte ovariectomisée présente un réseau de trabécules élargies. Ces photograhies attestent de la présence d'ostéoporose sur le fémur de la ratte ovariectomisée.

Sur la figure 5, la photographie de gauche présente la coupe d'un condyle fémoral d'une ratte ovariectomisée sur lequel a été inséré l'implant de l'exemple 2 depuis 90 jours et la photographie de droite présente la coupe d'un condyle fémoral d'une ratte ovariectomisée sur lequel a été inséré l'implant de l'exemple 3 depuis 90 jours. Macroscopiquement, tous les implants sont bien tolérés par le tissu osseux, on note l'absence de nécrose tissulaire.

Au bout de 90 jours l'implant de l'exemple 2 a pratiquement disparu : il a été dégradé au profit de la formation d'un nouvel os, cet implant présente une très bonne ostéointégration et assure la formation de tissus minéralisés propres à l'os mature. Cet implant pourrait présenter un avantage chez des jeunes sujets où le métabolisme osseux est très actif et chez lesquels le remodelage osseux est relativelment rapide.

Au bout de 90 jours, c'est-à dire au terme de l'étude, on observe encore nettement l'implant de l'exemple 3. La formation du nouvel os se poursuit plus lentement.

La figure 6 présentant une comparaison entre les analyses histologiques d'un implant selon l'exemple 3 (à droite) c'est-à-dire groupe e *vs* un implant comparatif selon l'exemple 4 sans chitosan (à gauche) c'est-à-dire groupe f. Ces photographies correspondent à un grossissement X50. On observe que l'intégration de l'implant selon l'invention dans l'os est plus importante que l'intégration de l'implant comparatif dans l'os.

### 2.4 Conclusions

Les implants selon l'invention sont biocompatibles et bien tolérés par l'organisme. En outre il a été observé que la poudre de co-produit de poissons et le chitosan sont intimement liés au moment de l'implantation, le chitosan étant ensuite libéré au niveau local. Le chitosan est donc ainsi disponible pour participer au traitement de l'ostéoporose, notamment induite par ovariéctomie.

Il a également été observé que les implants selon l'invention permettent la croissance d'une apatite de composition proche de celle de l'os sain ainsi qu'une maturation osseuse progressive.

## Revendications

1. [Biomatériau **caractérisé en ce qu'**il comprend, par rapport au poids total du biomatériau, de 70 à 90 %, de préférence de 80 à 85%, en poids d'une poudre d'au moins un co-produit de poissons choisi dans le groupe formé par les écailles de poissons et les arêtes de poissons et de 10 à 30 %, de préférence de 15 à 20%, en poids de chitosan.

2. Biomatériau selon la revendication 1 dans lequel le co-produit de poissons comprend du calcium et du phosphore selon un rapport calcium/phosphore en poids allant de 1,8 à 2,5 et de préférence allant de 2,0 à 2,5.

3. Biomatériau selon l'une quelconque des revendications précédentes dans lequel le co-produit de poissons comprend du collagène en une quantité allant de 20% à 40 %, et de préférence allant de 20% à 35%, en poids par rapport au poids total du co-produit de poissons.

4. Biomatériau selon l'une quelconque des revendications précédentes dans lequel le co-produit de poissons comprend du magnésium en une quantité allant de 0,30% à 0,60%, et de préférence allant de 0,44% à 0,46%, en poids par rapport au poids total du co-produit de poissons.

5. Biomatériau selon l'une quelconque des revendications précédentes dans lequel le co-produit de poissons comprend les éléments suivants en poids par rapport au poids total du co-produit de poissons : du zinc en une quantité allant de 0,010% à 0,011% ; du strontium en une quantité allant de 0,032% à 0,033 %.

6. Biomatériau selon l'une quelconque des revendications précédentes dans lequel la poudre de co-produit de poisson présente une granulométrie allant de 40 à 315 microns.

7. Procédé de fabrication du biomatériau selon l'une quelconque des revendications précédentes **caractérisé en ce qu'**il comprend les étapes suivantes, dans cet ordre :
- disposer d'une poudre d'au moins un co-produit de poissons choisi dans le groupe formé par les écailles de poissons et les arêtes de poissons, ladite poudre présentant une granulométrie allant de 40 à 315 microns ;
- disposer de chitosan ;
- préparer un mélange comprenant de 70 à 90 %, de préférence de 80 à 85%, en poids de poudre d'au moins un co-produit de poissons et de 10 à 30 % en poids, de préférence de 15 à 20% en poids de chitosan,
- agiter le mélange ;
- sécher le précipité obtenu ;
- récupérer le biomatériau obtenu.

8. Procédé de fabrication du biomatériau selon la revendication précédente dans laquelle la poudre de co-produit de poissons est obtenue en mettant en oeuvre les étapes suivantes, dans cet ordre :
- disposer d'au moins un co-produit de poissons choisi parmi les écailles de poissons et les arêtes de poissons ;
- placer le co-produit de poissons dans un bain d'eau à une température allant de 50°C à 100°C pendant une durée allant de 5 à 120 minutes;
- sécher le co-produit ;
- broyer le co-produit de manière apte à obtenir une poudre de granulométrie allant de 40 à 315 microns ;
- récupérer la poudre de co-produit de poissons obtenue.

9. Composition comprenant un biomatériau selon l'une quelconque des revendications 1 à 6 ou obtenu selon l'un des revendications 7 ou 8 et au moins un agent choisi parmi les médicaments en particulier les antibiotiques, les bisphosphonates, les polymères thermoplastiques en particulier choisis parmi l'acide polylactique, l'acide poly (lactique-co-glycolique), le polybutylène succinate, les polyhydroxyalcanoates, la polycaprolactone, le polybutylène-co-adipate téréphtalate.

10. Implant comprenant un biomatériau selon l'une quelconque des revendications 1 à 6 ou obtenu par le procédé selon l'une quelconque des revendications 7 à 8 ou comprenant la composition selon la revendication 9.

11. Implant selon la revendication 10 comprenant, en poids par rapport au poids total de l'implant, de 90 à 100 % d'un biomatériau selon l'une quelconque des revendications 1 à 6 ou obtenu par le procédé selon l'une quelconque des revendications 7 à 8 et de 0 à 10 % d'au moins un agent choisi parmi les bisphosphonates et les médicaments, en particulier les antibiotiques.

12. Implant selon la revendication 10 comprenant en poids par rapport au poids total de l'implant, de 30 à 50 % d'un biomatériau selon l'une quelconque des revendications 1 à 6 ou obtenu par le procédé selon l'une quelconque des revendications 7 à 8, de 50 à 70% d'un matériau thermoplastique choisi dans le groupe formé par l'acide polylactique, l'acide poly (lactique-co-glycolique), le polybutylène succinate, les polyhydroxyalcanoates, la polycaprolactone, le polybutylène-co-adipate téréphtalate et de 0 à 10 % d'au moins un agent choisi parmi les bisphosphonates et les médicaments, en particulier les antibiotiques.

13. Implant selon l'une quelconque des revendications 10 à 12 pour son utilisation pour la repousse osseuse, la reconstruction osseuse, et/ou le traitement de l'ostéoporose.

14. Procédé de fabrication de l'implant selon la revendication 12 comprenant les étapes suivantes, dans cet ordre,
- mélange du biomatériau selon l'une quelconque des revendications 1 à 6, avec au moins un matériau thermoplastique choisi parmi l'acide polylactique, l'acide poly (lactique-co-glycolique), le polybutylène succinate, les polyhydroxyalcanoates, la polycaprolactone, le polybutylène-co-adipate téréphtalate ; au moins un agent porogène et éventuellement au moins un agent choisi parmi les médicaments en particulier les antibiotiques, les bisphosphonates ;
- mise en forme du mélange par impression 3D par dépôt de fil fondu,
- élimination des agents porogènes de préférence par lixiviation,
- éventuellement séchage de l'implant obtenu.]
